# EUROPEAN PATENT APPLICATION

(11) **EP 3 178 455 A1**
(43) Date of publication of application: **14.06.2017**
(21) Application number: 14899274.6
(22) Date of filing: 04.08.2014
(51) Int. Cl.: A61F 13/15, A61K 8/02, A61K 8/92, A61K 8/97, A61L 15/34, A61L 15/46, A61Q 19/00

(54) **DISPOSABLE ABSORBENT ITEM COMPRISING AN ANTI-CHAFING FORMULA BASED ON NATURAL COMPONENTS**

(71) Applicant: Grupo P.I. Mabe, S.A. de C.V., 72230 Puebla (MX)
(72) Inventor: FAJARDO ESLAVA, Bernardo, 72230 Puebla (MX); MARTINI MERLO, Juan Miguel, 72230 Puebla (MX); CANALES ESPINOSA DE LOS MONTEROS, Carlos, 72230 Puebla (MX); SÁNCHEZ FERNÁNDEZ, Lucía del Carmen, 72230 Puebla (MX)
(74) Representative: Gil-Vega, Victor
(86) International application number: PCT/IB2014/063691
(87) International publication number: WO 2016/020721

(57) **Abstract**

The present invention protects a disposable hygienic article characterized in that it has a formulation that promotes the skin care through the use of natural compounds such as the cane wax extract, which has pharmacological properties such as healing and anti-inflammatory, the formulation may also contain agents that helps neutralize unpleasant odors such as chlorophyll, an antimicrobial agent such as grapefruit seeds extract and a fragrance.

## Description

The disposable hygienic products such as disposable diapers for babies, disposable diapers for adults, training pants, articles for incontinents, feminine pads, pantiliners and other similar articles, are articles aimed to absorb and contain the bodily exudates and to be discarded after its use. There are also hygienic products aimed for the hygiene of the intimate area, urogenital areas and skin in general such as the wet wipes. There are many proposals aiming to improve the characteristics of this kind of products, some focused on improving the absorption and retention, others on eliminating leaks, some others on making the articles soft, comfortable and discreet for the user, others on reducing costs, etc.

Another branch of research to improve this type of articles focuses on the protection of the skin of the user, in order to prevent rashes and keep the skin healthy and lubricated.

For many years there have been attempts to incorporate different types of lotions to the disposable hygienic products to improve the conditions of the skin during their use, examples of these attempts are the United States Patents 3,585,998 by Hayford et al; US 3,464,413 by Goldfarb et al; US 3,896,807 by Buchalter; US 3,489,148 by Duncan et al and many more that are not mentioned, these formulations have not been efficient enough, therefore alternatives that actually protect the skin of the user have been sought. Other more recent examples of formulations used for this purpose are the United States Patents US 5,693,037 applied by Procter & Gamble, which protects the use of a silicone-based surfactant; US 5,830,487 applied by Procter & Gamble which protects a lotion containing an organic acid such as citric acid and hydrophilic solvents such as propylene glycol so that it kills the viruses and provides softness to the top layer; US 5,869,033 applied by EnviroDerm Pharmaceuticals, protects a fabric which incorporates an organoleptic clay dispersed preferably in a superabsorbent matrix; US 5,938,649 to Drypers Corp., protects the use of aloe vera in the top layer of a disposable absorbent article; US 6,287,581 applied by Kimberly-Clark protects the use, in the surface of an article of this type, of a lipid-enriched hydrophobic composition, comprising a natural oil, a sterol, a surfactant, a moisturizer and an emollient, as well as many other patents not cited herein.

The present invention proposes a naturally derived formulation to be applied in a disposable hygienic product that will contact the skin of the user for its protection, it also helps in the skin regeneration and in the control of unpleasant odors; the formulation contains emollients and naturally derived odor control agents. Cane wax extract is used; it has pharmacological properties recognized as anti-inflammatory and wound healing. Chlorophyll or its derivatives are used as odor control agent. On the other hand, it is considered the addition of an antimicrobial agent such as grapefruit seeds extract to the formulation.

### Objectives of the Invention

An objective of the present invention is the use of a formulation to protect the skin of the user during the use of disposable hygienic products such as disposable diapers, feminine pads, pantiliners, wet wipes and articles for incontinence, which will also help in the dermal regeneration (wound healing) more efficiently than the emollients currently used in these products such as vitamin E, chamomile extract or any other naturally derived anti-inflammatory ingredients.

Another objective of the invention is that said formulation preferably has naturally derived components.

As an additional objective it can be considered the inclusion of an agent which helps control the unpleasant odors generated by the bodily exudates, and that the formulation also helps to inhibit the growth of micro-organisms in the article.

### Detailed Description of the Invention

The disposable hygienic products are articles that are used in contact with the body, whether to receive and contain the exudates thereof or for general cleaning, among them we find disposable diapers for babies, training pants, feminine pads, pantiliners, wet wipes and incontinence articles. It is desirable that this type of articles help protect the skin of the user, since the constant rubbing of the skin with the top layer of some products and the contact of the skin with moisture and feces may cause damage and discomfort to the user. There are infinity of proposals focused on the protection of the skin of the user through the use of lotions containing different compounds that soften the top layer and lubricate the skin, however none of these involves the use of naturally derived elements mixed in a solution so that they can act together to lubricate and efficiently regenerate the skin, as well as to control the unpleasant odors which are characteristic of the bodily exudates.

The present invention protects the use of cane wax extract in a solution that may also contain a naturally derived component to control odors such as chlorophyll or its derivatives and an anti-microbial agent such as grapefruit seeds extract, so that the solution is applied in the disposable hygienic product in order to protect the skin of the user, help in its regeneration in case of wounds, control unpleasant odors and inhibit microbial development.

The cane wax extract is recognized by its pharmacological properties as wound healing, anti-inflammatory and antimicrobial.

The cane wax extract helps to accelerate the dermis keratinization and increases the moisture level in the skin leaving the skin smooth and supple.

Said extract is obtained as a by-product in the production of sugar cane, it is a uniform liquid, with a soft green color and with a characteristic odor and is composed of a mixture of fatty acids (myristic, palmitic, stearic, arachidic, dodecanoic acids), higher alcohols (tetracosanol, hexacosanol, octacosanol and triacosanol) and phytosterols (stigmasterol, campesterol and beta-citosterol) among others.

In the proposed formulation, the cane wax extract or its derivatives are efficient when used in amounts from 1.0% by weight or more.

The chlorophyll or its derivatives such as copper, sodium or potassium chlorophyll, are natural adsorbents, and, through Van der Waals forces, they attract the compounds that generate unpleasant odors, said compounds adhere to the chlorophyll molecule being trapped therein, thus the chlorophyll and its derivatives have been recognized for being powerful deodorants, so that, when used together with natural extracts such as the cane wax extract, a solution which, besides helping to lubricate and regenerate the skin, neutralizes the unpleasant odors, is obtained.

In the proposed formulation, the chlorophyll or its derivatives are efficient when used in amounts that range from 0.01 % by weight.

The antimicrobial agent such as the grapefruit seed extract has antimicrobial activity against most Gram-positive bacteria, Gram-negative bacteria, fungi and yeasts, another additional feature is its anti-inflammatory activity as it has proven to be a powerful cycle-oxygenases and lipoxygenases inhibitor, enzymatic keys of the arachidonic acid metabolism, resulting in a reduced synthesis of pro-inflammatory metabolites.

In the proposed formulation, the antimicrobial agent obtained from grapefruit seeds extract is efficient when used in amounts from 0.01 % by weight or more.

As carrier for the solution it may be used a pharmaceutical grade propylene glycols (USP) such as mono-propylene glycol instead of water, in order to avoid the growth of microorganisms.

According to the information above, the formulation proposed to be applied in disposable hygienic products contains at least 1.0% by weight of cane wax extract and at least 1% by weight of a carrier. Also, the formulation may have an odor control agent such as chlorophyll or its derivatives in a concentration in amounts ranging from 0.01% by weight and of an antimicrobial agent, such as grapefruit seeds extract, in amounts ranging from 0.01% by weight.

Generally, to this kind of articles is added a fragrance to make them more attractive for the consumer, this may be contained in the proposed formulation in amounts ranging from 0.01% by weight, in this way the emollient and the fragrance are added to the product in a single operation.

The proposed formulation is applied in the disposable hygienic products in quantities ranging from 0.06 g / cm².

### Examples

The proposed formulation was experimentally tested under the following conditions and with the following results:

**Anti-Rash Formulation with Healing Effect Proposal**

| Compound | Minimum Percentage (%) |
|---|---|
| Cane Wax Extract | 5.00 |
| Chlorophyll or its derivatives | 0.05 |
| Grapefruit seed extract | 0.5 |
| Mono Propylene Glycol | 94.45 |

### Experimental development

This formulation was tested using the experimental method 240416-3 CIDEM, IPN 1994 which comprises the use of 30 male Swiss guinea pigs housed in individual cages for 21 days with controlled temperature of 22 ± 2°C, 40-60% humidity and 12/12 h light/dark cycle.

All animals were shaved 24 h before performing the cut in the scapular region, in a 2 x 2 cm2 area which included skin and subcutaneous cellular tissue.

The application of the emollient to be evaluated was performed daily during a period of 21 days, time in which it was observed the formation and falling of the scab and healing of the area during the entire experimental period.

### Conclusion

It may be concluded that the emollient with 5% cane wax extract was effective in healing wounds developed in animals for experimentation because, after four days of testing, 80% of the animals had already formed a scab and 90% had healed after 21 days, while the placebo samples (only carrier), showed only 10% of the animals had formed scabs after four days, and 10% of the animals had healed after 21 days.

With the placebo sample without extract or carrier, 0% of the animals had formed scabs after four days, and 20% of the animals had healed after 21 days.

Home Use Test were performed whereby 50 users of the size 4 economic segment (babies with rashes) were contacted, 25 users tested the Sample A which contains the proposed healing formulation and the remaining 25 tested the Sample B as control sample; 10 diapers were delivered to each participant, explaining the dynamics of the study.

| **RESULTS OF THE HOME TEST USE** | | |
|---|---|---|
| **-Overall assessment of the product-** | | |
| (% regarding 25 users) | **A** | **B** |
| Very Good | 4/4 | 0/0 |
| Good | 44/60 | 40/60 |
| Regular | 52/36 | 60/40 |
| Poor | 0 | 0 |
| Very Poor | 0 | 0 |

| **-Functional characteristics evaluation-** | | | | |
|---|---|---|---|---|
| | A | | B | |
| **Leaks presence (p)** | 1.25 | | 1.46 | |
| **Dry skin at daytime** | 72 | | 72 | |
| **Dry skin at nighttime** | 44 | | 4.0 | |
| **Fitting Score** | | | | |
| **Tight** | 0 | | 0 | |
| **Well** | 100 | | 100 | |
| **Loose** | 0 | | 0 | |
| **Covers Properly** | 100 | | 98 | |
| **Proper function of the tabs** | 100 | | 100 | |
| **Works at Night** | 100 | | 100 | |
| **Rating** | 8.96 + .84 | 9 + .82 | 8.52 + .65 | 8.64 + .7 |
| **The Irritation was Reduced** | 88 | 92 | 12 | 16 |
| **Little** | 56 | 20 | 12 | 12 |
| **A lot** | 32 | 40 | 0 | 0 |
| **Total** | 0 | 32 | 0 | 0 |
| **Nothing** | 12 | 8 | 88 | 88 |

From the Home Use Test study, it may be seen that sample A has a very high ratio with respect to the sample B regarding the reduction of the irritation, in the first and second delivery of the sample. While sample B in the two deliveries has the same reduction of irritation, lower than the value of the sample A.

| **- Remarks -** | | |
|---|---|---|
| **Users** | A | B |
| **Does not Irritate** | 2 | 5 |
| **Irritation was Reduced** | 9 | 1 |
| Works well | 6 | 1 |
| Dry skin | 1 | |
| No Leaks | 7 | 6 |
| Last | 10 | 3 |
| Good tabs | 1 | |
| Is not deformed | 1 | 2 |
| Fit | 1 | 4 |
| Irritation did not increase | | 2 |
| Irritates | | 1 |
| Less Leaks | | 1 |
| Works at Night | | 2 |

While the invention has been claimed based on a currently preferred embodiment, it is clear that several changes and modifications may be made to the same, such changes and modifications are within the scope of the invention as defined in the following claims.

## Claims

1. A disposable absorbent article basically comprised by an inner cover, an outer cover and an absorbent core there between, comprising a cane wax extract based dermal protective and regenerative formulation.

2. The disposable absorbent article according to claim 1, wherein the formulation is applied on the inner cover.

3. The disposable absorbent article according to claim 1, wherein the formulation is applied on the absorbent core.

4. A disposable hygienic article basically comprised by a non-woven fabric or cellulose fibers layer, wherein it is coated with a cane wax extract based dermal protective and regenerative formulation.

5. The disposable article according to claims 1, 2, 3 and 4, wherein the cane wax extract is present in the formulation at a concentration of at least 1% by weight.

6. The disposable article according to claims 1, 2, 3 and 4, wherein the formulation with cane wax extract is applied in amounts ranging from 0.06 g / cm².

7. The disposable article as claimed in claims 1, 2, 3 and 4, wherein the formulation contains an unpleasant odor neutralizing agent such as chlorophyll or derivatives thereof.

8. The disposable absorbent article as claimed in claim 7, wherein the unpleasant odor neutralizing agent such as chlorophyll or derivatives thereof, is present in the formulation in amounts ranging from 0.01 to 10.0% by weight.

9. The disposable absorbent article as claimed in claim 7, wherein copper, sodium or potassium chlorophyll is used as a chlorophyll derivative.

10. The disposable absorbent article as claimed in claims 1, 2, 3 and 4, wherein the formulation contains an antimicrobial agent such as grapefruit seed extract.

11. The disposable absorbent article as claimed in claim 10, wherein the antimicrobial agent is present in the formulation in amounts ranging from 0.01 to 10.0% by weight.

12. The disposable absorbent article as claimed in claims 1, 2, 3 and 4, wherein the formulation contains a fragrance.

13. The disposable absorbent article as claimed in claim 12, wherein the fragrance is present in the formulation in amounts ranging from 0.01 to 10.0% by weight.

14. The disposable absorbent article as claimed in claims 1, 2, 3 and 4, wherein the formulation contains a carrier in amounts ranging from 1% by weight.

15. The disposable absorbent article as claimed in claim 14, wherein the carrier used in the formulation is comprised by pharmaceutical grade propylene glycols.

16. The disposable absorbent article as claimed in claim 15, wherein the carrier used in the formulation is UPS mono propylene glycol.
